# EUROPEAN PATENT SPECIFICATION

(11) **EP 2 992 929 B1**
(45) Date of publication and mention of the grant of the patent: **01.11.2017**
(21) Application number: 14003041.2
(22) Date of filing: 03.09.2014
(51) Int. Cl.: A61N 5/06, A61N 1/36, A61N 5/067, A61N 1/04

(54) **Treatment apparatus with a positioning device and a stimulation device**
Behandlungsvorrichtung mit Positionierungsvorrichtung und Stimulationsvorrichtung
Appareil de traitement avec un dispositif de positionnement et un dispositif de stimulation

(43) Date of publication of application: 09.03.2016
(73) Proprietor: GMG Beratungs- und Beteiligungs Verwaltungs GmbH, 82049 Pullach (DE)
(72) Inventor: Teichert, Klaus, Dover, CT17 0TF (GB)
(74) Representative: Beier, Ralph

(56) References cited:
- DE-A1-102004 018 340
- US-A1- 2006 173 514

## Description

### TECHNICAL FIELD

The present invention relates to a positioning device, which is adapted for positioning a stimulation device on a body surface of an organism, e. g. a person or animal to be treated. Furthermore, the present invention relates to a treatment apparatus comprising the positioning device and a stimulation device being coupled with the positioning device. Furthermore, the present invention relates to a stimulation system, comprising a plurality of treatment apparatuses and a main control. Applications of the invention are available in particular in the field of stimulation treatments, using electrical and/or optical sources.

### TECHNICAL BACKGROUND

It is generally known to locally stimulate a body of a person to be treated by illuminating and/or heating the body surface with one or more light sources, e. g. laser diodes, or for improving wound healing. Light sources emitting ultraviolet, visible or infrared light are used for the exposure of biological tissue to radiation. For example, methods for exposing tissue to laser light have been developed which are known by the name of "Low Level Laser Therapy" (LLLT). LLLT methods are in particular based on stimulating materials-related processes in cells or tissue by the admission of light and/or heat (see e. g. US 5 464 436 or US 2006/0173514 A1).

Furthermore, the body can be stimulated by applying an electrical current. For example, DE 202 07 955 U1 discloses a stimulation device for electrically stimulating the body surface, wherein an electrical stimulation unit is adherently coupled with the body surface. The electrical stimulation unit is connected via a supply cable with a power supply. This technique has disadvantages if multiple electrical stimulation units are to be positioned on the body surface as the plurality of supply cables disturb the stimulation treatment. Furthermore, the supply cables have a certain weight, thus requiring a strong adherent connection between the electrical stimulation unit and the body surface. Again, the strong adherent connection causes an uncomfortable feeling for the person, thus impairing the stimulation result.

DE 101 28 629 A1 discloses a skin plaster, which carries a light source, a power supply and a control unit. The skin plaster is adapted for improving wound healing by optical stimulation. The skin plaster has an advantage as it does not require a connection with a main control via supply cables, thus allowing a comfortable treatment of the body surface. However, the skin plaster has a disadvantage in terms of controlling the operation thereof. As an example, if multiple skin plasters would be required, a synchronized switching of the light sources would not be possible with the conventional device. As a further disadvantage, the skin plaster of DE 101 28 629 A1 has a complex and expensive structure.

DE 10 2004 018 340 A1 disclose a treatment device for an external stimulation of a body, which is adapted for a wireless control. The treatment device comprises a stimulation device for an optical stimulation of the body surface and a positioning device for adherently positioning the stimulation device on the body surface. The positioning device includes an antenna, which receives control signals for controlling the stimulation device. The antenna is electrically connected with a control unit included in the stimulation device. Advantageously, a plurality of the conventional treatment devices can be simultaneously switched on using a radio control signal, which is received by the antennas and submitted to the control units of all treatment devices. Furthermore, different treatment devices can be controlled with different control signals, e.g. for applying specific stimulation programmes. However, the practical application of the conventional treatment device may suffer from the following disadvantages and restrictions. Firstly, there is an interest in minimizing the size of the treatment device for facilitating the adherent fixing thereof on the body surface. However, this is restricted due to the multiple components included in the stimulation device, like e.g. the control unit. Furthermore, the submission of the control signals received by the antenna via an electrical contact to the stimulation device is prone to be disturbed, e.g. by contact failures. As a further disadvantage, it has been found that an interference of the control signals of different treatment devices can occur. Finally, the structure and costs of manufacturing the conventional treatment device may result in restrictions for a mass application thereof.

### OBJECTIVE OF THE INVENTION

It is a first objective of the invention to provide an improved positioning device, which is adapted for positioning a stimulation device and which is capable of avoiding disadvantages of conventional techniques. In particular, it is the first objective of the invention to provide the positioning device with improved reliability of the electrical connection with the stimulation device and an improved reliability of adherently fixing the stimulation device on a body surface (e. g. skin of a person). It is a further objective of the invention to provide an improved treatment apparatus, in particular for LLLT applications, which is capable of avoiding disadvantages of conventional techniques and which in particular has an improved operation reliability. It is a further objective of the invention to provide an improved stimulation system comprising a plurality of treatment apparatuses.

These objectives are solved by a positioning device, a treatment apparatus, and a stimulation system comprising the features of the independent claims, respectively. Preferred embodiments and applications of the invention are defined in the dependent claims.

### SUMMARY OF THE INVENTION

According to a first general aspect of the invention, the above objective is solved by a positioning device (holder, applicator), which is adapted for adherently fixing a stimulation device on a body surface of an organism, wherein the positioning device comprises an adhering component, an antenna device and a carrier contact section. The adhering component generally is adapted for accommodating the stimulation device in use and for a releasable arrangement with the stimulation device on the body surface. Preferably, the adhering component is a flexible carrier being capable of forming a receptacle, wherein the stimulation device can be arranged at the receptacle. The stimulation device can be hold by the adhering component, in particular in the adherently fixed condition thereof on a body surface. The antenna device is fixedly connected with the adhering component, e.g. it is integrated into the material of the adhering component or fixed to a surface thereof. The antenna device is adapted for receiving control signals from a main control for controlling the stimulation device, when it is coupled with the positioning device. The carrier contact section is a further portion of the adhering component. It is arranged such that an electrical contact is provided with the stimulation device when it is coupled with the positioning device.

According to the invention, the positioning device further comprises a switching device, which is fixedly connected with the adhering component. The switching device is electrically connected with the antenna device and the carrier contact section, and it is adapted for fulfilling a switching function in response to a control signal received by the antenna device. The switching device is electrically interconnected between the antenna device and the carrier contact section. Thus, contrary to the conventional technique, e.g. according to DE 10 2004 018 340 A1, switching signals are applied to the carrier contact section rather than radio frequency signals. The switching signals are less sensitive against contact failures or other wireless control signals. Furthermore, the provision of the switching device allows a simplified structure of the stimulation device. The size and weight of the stimulation device can be minimized, thus allowing a fixation on the body surface with increased reliability. According to second general aspect of the invention, the above objective is solved by a treatment apparatus comprising the positioning device according to the above first general aspect of the invention and a stimulation device, in particular according to the fourth general aspect of the invention cited below, wherein the stimulation device is coupled with the positioning device such that a stimulation device contact section of the stimulation device is in electrical contact with the carrier contact section of the positioning device. The stimulation device is detachably coupled with the positioning device, so that a separation of both components after use is possible.

According to a third general aspect of the invention, the above objective is solved by a stimulation system, which comprises a plurality of treatment apparatuses according to the second general aspect of the invention and a main control, which is configured for creating and transmitting control signals for controlling the stimulation devices of the treatment apparatuses, respectively.

Furthermore, a stimulation device (in particular low level laser therapy device) is described, which comprises at least one stimulation unit and a power supply section for supplying electrical power to the at least one stimulation unit. The stimulation device further comprises a stimulation device contact section, which is arranged, e.g. exposed, on an outer surface of the stimulation device and which is adapted for receiving a
switching signal. The stimulation device contact section is electrically connected with the at least one stimulation unit and the power supply section such that the at least one stimulation unit can be directly switched on or off in response to a switching signal applied to the stimulation device contact section, e. g. according to a predetermined stimulation pattern. The stimulation device does not include a control unit for converting control signals received via an antenna into switching signals. Accordingly, the structure of the stimulation device is simplified and the size and weight thereof can be minimized.

According to a preferred embodiment of the invention, the adhering component of the inventive positioning device comprises at least one, preferably at least two adhesive layers, which are exposed for a contact with the body surface. Furthermore, the adhering component preferably comprises a carrier web, which is arranged adjacent to or between the one or more adhesive layer(s) wherein the carrier web carries the antenna device and the switching device and further is adapted for at least partially enclosing the stimulation device. Thus, in combination with the body surface, the carrier web is adapted to hold the stimulation device in use.

According to a particularly preferred embodiment of the invention, the antenna device and the switching device are arranged on an outer surface of the carrier web, while the carrier contact section is arranged and exposed on an inner side of the carrier web, i.e. on an inner side of the receptacle provided by the carrier web, facing to the stimulation device. Accordingly, the transmission of control signals to the antenna device is improved. Furthermore, an optical access to the switching device is allowed. If the switching device is adapted with an indicator light, an operation state of the switching device can be monitored by a user.

According to a further particularly preferred embodiment of the inventive positioning device, the antenna device, the switching device and the carrier contact section are arranged such that the distance of an electrical connection between the antenna device and the switching device is minimized. In particular, if the stimulation device is coupled with the positioning device, the length of the electrical connection between the antenna device and the switching device is shorter than the length of an electrical connection between the switching device via the carrier contact section to the stimulation device. Advantageously, the transmission of control signals via electrical connections within the positioning device, which could be deteriorated by other radio control signals, is minimized. Accordingly, the risk of interferences can be reduced. The carrier contact section includes two contacts, which are to be connected with associated contacts of the stimulation device contact section. The switching device can be adapted for a pulsed operation, so that the stimulation device can be switched with pulse shaped switching signals, e. g. having frequencies in Hz- to kHz-ranges. The switching device is adapted for electrically connecting the contacts in response to a control signal received by the antenna device. The contacts of the carrier contact section are shortcircuited by the switching device in dependency on the control signal. In other words, the stimulation device can be directly switched with the switching device by closing an electrical circuit including the at least one stimulation unit and the power supply section. Alternatively, switching signals of the switching device can be submitted via the contacts for activating the stimulation device.

According to a further advantageous embodiment, the adhering component is a disposable component. The adhering component can be disposed as scrap material after use, while the stimulation device can be reused, in particular for a certain number of operation cycles, e.g. for 20 operation and recharging cycles. With a particularly preferred embodiment of the invention, the adhering component is an adhesive plaster with two adhesive layers with the carrier web there between. Advantageously, the inventive treatment apparatus including the stimulation device coupled with the positioning device simply can be applied to the body surface, like a conventional plaster.

According to a preferred embodiment of the inventive treatment device, the at least one stimulation unit includes at least one of an optical stimulation unit and an electrical stimulation unit. Preferably, both of the optical and electrical stimulation units are included in the stimulation device. With a treatment method, both of the optical and electrical stimulation units are driven simultaneously, so that the person feels the application of an electrical stimulation current simultaneously with the application of an optical stimulation.

According to a particularly preferred embodiment of the invention, the stimulation device of the treatment apparatus is adapted for an output of a
stimulation having the effect of Low Level Laser Therapy. Accordingly, the at least one stimulation unit includes at least the optical stimulation unit, including one or more laser diodes, emitting monochromatic light in the visible, near-infrared or infrared range, in particular in a range from 600 nm to 1000 nm. The optical stimulation unit is exposed on a surface of the stimulation device, so that it can be arranged in contact with a body surface. Preferably, the optical stimulation unit is adapted for an output power below 500 mW, in particular below 300 mW, e. g. 100 mW or lower, like 50 mW. The illuminated area of the surface to be treated is e. g. 1 cm² or smaller. Practical power densities are e. g. 25 mW/cm² or lower, like 10 mW/cm² or 5 mW/cm².

According to a further preferred embodiment of the inventive treatment apparatus, an enclosure (housing) of the stimulation device is provided, which accommodates the at least one stimulation unit and the power supply. Preferably, the enclosure includes these components in a sealed, in particular humidity tight fashion. On one side of the enclosure (bottom surface), the at least one stimulation unit, in particular the optical stimulation unit and the electrical stimulation unit, is/are exposed towards the surrounding, while on a second, preferably opposite side of the enclosure (upper surface), the stimulation device contact section is arranged for a contact with the carrier contact section of the positioning device. Advantageously, the electrical connection between both contact sections reliably can be closed simply by the step of fixing the stimulation device with the positioning device on the body surface.

Further advantages of the invention are obtained if the stimulation device includes an anchoring section, which is adapted for a releasable connection with the positioning device. Advantageously, the anchoring section improves the reliability of the mechanical connection of the stimulation device with the positioning device and thus the reliability of the electrical connection there between. Preferably, the anchoring section comprises spikes and/or receptacle grooves, which are arranged for an engagement with the carrier web of the positioning device. Preferably, the spikes and/or receptacle grooves are provided on an outer side of the stimulation device enclosure.

Advantageously, the stimulation device contact section is adapted for fulfilling a double function. Firstly, it is electrically connected with the carrier contact section for the switching operation of the at least one stimulation unit. Secondly, the stimulation device contact section can be used for recharging the power supply section in the stimulation device. To this end, preferably the main control or an additional recharging device has at least one interface, preferably a plurality of interfaces, for electrically coupling one or more stimulation device(s) and recharging thereof. The recharging current can be supplied to the power supply via the at least one stimulation unit or, or it can be bypassed by a circuitry within the stimulation device.

### BRIEF DESCRIPTION OF THE DRAWINGS

Further advantages and details of the invention are described in the following with reference to the attached drawings, which show in:
- Figure 1:: perspective views of a preferred embodiment of a positioning device according to the invention;
- Figure 2:: a side view of a preferred embodiment of a treatment apparatus according to the invention;
- Figure 3:: various outer views of the stimulation device, included in a treatment apparatus according to the invention;
- Figure 4:: various views of a further stimulation device, included in a treatment apparatus according to the invention;
- Figure 5:: illustrations of the internal structure of a stimulation device, included in a treatment apparatus according to the invention;
- Figure 6:: an example of a main control of a stimulation system according to the invention; and
- Figure 7:: an example of a recharging device of a stimulation system according to the invention.

### PREFERRED EMBODIMENTS OF THE INVENTION

Embodiments of the invention are described in the following with particular reference to the application of a stimulation device including both an electrical stimulation unit and an optical stimulation unit. It is emphasized that the implementation of the invention is not restricted to the illustrated embodiments but rather possible also with stimulation devices including an electrical stimulation unit or an optical stimulation unit only. Details of stimulation methods, in particular treatment procedures with predetermined electrical and/or optical stimulation patterns comprising pulse stimulation currents and/or pulsed stimulation illumination are not described as they are known as such from prior art. Preferably, LLLT treatment procedures are applied, wherein the optical stimulation patterns is switched according to a known LLLT protocol. If the optical and electrical stimulation are combined, both of the optical and electrical stimulation units preferably are operated in synchronized fashion, i. e. they are simultaneously switched on/off. The drawings show schematic illustrations only. In practice, the details of the illustrated components can be modified and adapted to the requirements of the practical use.

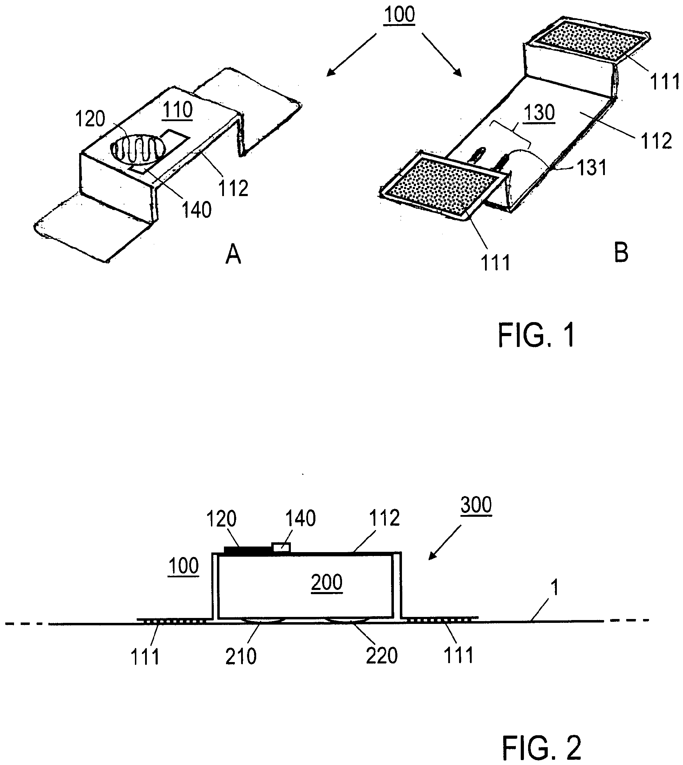

Figure 1 schematically illustrates a preferred embodiment of an inventive positioning device 100, comprising the adhering component 110, the antenna device 120, the carrier contact section 130 and the switching device 140. Figure 1A shows an upper perspective view of the positioning device 100, while Figure 1B illustrates the corresponding perspective view from below.

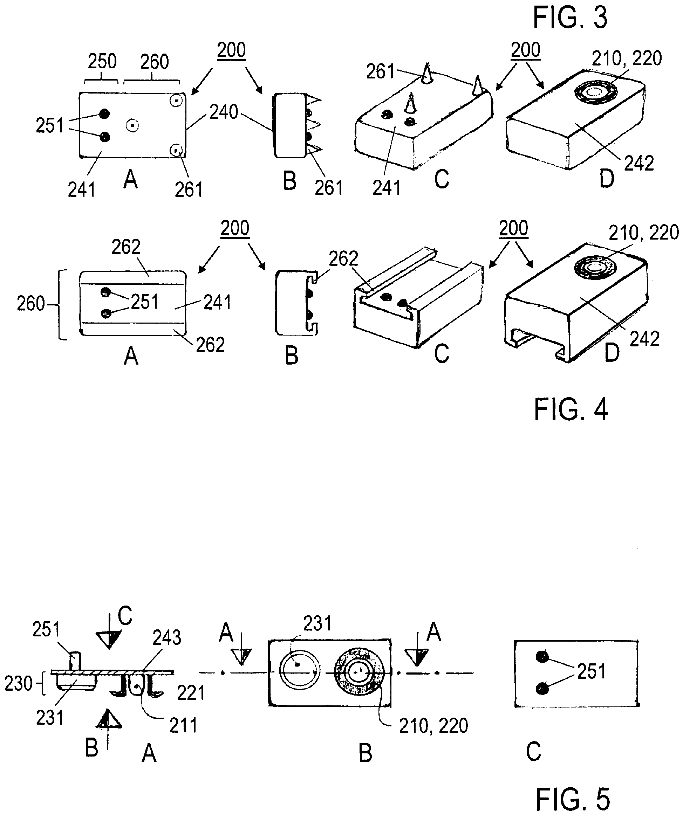

The adherent component 110 comprises a strip, which is made of a flexible material, e.g. a textile material and/or a plastics material. With a practical example, the strip has a rectangular shape with a size of e.g. 1 cm * 5 cm. Figures 1A and 1B illustrate the positioning device in a folded condition, thus emphasizing a receptacle formed by the strip for accommodating an stimulation device, e.g. according to Figures 3 or 4. Alternatively, without the coupling with the stimulation device, the strip of the adhering component 110 can have a plane shape. As a further alternative, the folded shape (as shown) can be created by the structure of the strip, e.g. by the provision of appropriate folding sections perpendicular to the longitudinal extension of the strip.

At the longitudinal ends of the adhering component 110, two wings are provided, each carrying an adhesive layer 111. The adhesive layers are exposed for a connection with a body surface 1 (see Figure 2). Furthermore, a central section of the strip provides a carrier web 112, which forms the receptacle for accommodating the stimulation device.

The antenna device 120 and the switching device 140 are arranged on an outer surface of the adhering component 110, in particular on the carrier web 112. Alternatively, they could be arranged on one of the wings carrying the adhesive layers 111. The antenna device 120 and the switching device 140 may comprise separate components, wherein the antenna device 120 comprises an antenna for receiving radio frequency control signals as it is known as such and the switching device 140 comprises a circuit for creating switching signals in response to the control signals, in particular for creating a short-circuit between the contacts 131 of the carrier contact section 130. Alternatively, both of the antenna device 120 and the switching device 140 are provided as a common component, e. g. as a transponder device including an antenna and an integrated circuit for fulfilling the switching function of the switching device 140.

The carrier contact section 130 comprises two contacts 131, being formed as straight strips on the inner side of the carrier web 112. The contacts 131 are directly connected with the switching device 140. The contacts 131 comprise e.g. Cu with a length of e.g. 5 mm and a width of e.g. 1 mm.

Figure 2 schematically shows a side view of an embodiment of the inventive treatment apparatus 300 comprising the positioning device 100 as shown in Figure 1 and the stimulation device 200, which is further described below with reference to Figures 3 to 5. The adhering component of the positioning device 100 is connected via the adhesive layers 111 with the body surface 1 of a person to be stimulated. On the upper side of the carrier web 112 of the adhering component, the antenna device 120 and the switching device 140 are arranged, e. g. side by side, while the stimulation device 200 is accommodated by the receptacle formed by the carrier web 112. With the fixed condition, the electrical stimulation unit 210 and the optical stimulation unit 220 point towards the body surface 1. Activating the stimulation units 210, 220 with the switching device 140 results in a stimulation of the body according to a predetermined stimulation protocol.

Figures 3A to 3D illustrate various outer views of the stimulation device 200. The stimulation device 200 comprises an enclosure 240 having an upper surface 241 (Figure 3A) and a bottom surface 242 (Figure 3D). The enclosure 240 is made of e.g. plastic or steel. Preferably, the enclosure 240 is sealed at all sides thereof so that an introduction of humidity or liquid water into the inner space of the enclosure 240 is prevented. The upper surface 241 includes the stimulation device contact section 250 with two contacts 251 being exposed to the carrier contact section 130 (see Figure 1B). Furthermore, the upper surface 241 carries an anchoring section 260 comprising three spikes 261 (see Figures 3B, 3C), which protrude from the upper surface 241. When the stimulation device 200 is coupled with the positioning device 100, spikes 261 are introduced into the carrier web 112 for a fixation of the stimulation device at the positioning device 100.

On the bottom surface 242, the electrical and optical stimulation units 210, 220 are exposed. With the embodiment illustrated in Figure 3D, both stimulation units have a concentric arrangement with an inner optical stimulation unit 210, e.g. a light emitting diode or a laser diode 211, and an outer stimulation electrode 221 (see Figure 5A). Alternatively, multiple laser diodes 211 and/or multiple stimulation electrodes 221 can be provided at a stimulation device 200.

Figure 4 shows outer views of a further stimulation device 200, in particular with the upper surface 241 and the bottom surface 242. The upper surface 241 includes the contacts 251 and an anchoring section 260 with two receptacle grooves 262 (see Figures 4B, 4C). The receptacle grooves 262 are configured for an accommodation of the edges of the carrier web 112 (see Figures 1, 2).

Figure 5 shows internal views of the stimulation device 200. According to Figure 5A, a support plate 243, like e.g. a printed circuit board, is arranged in the enclosure 240 (not shown in Figure 5). On a first side of the support plate 243, facing to the bottom surface of the enclosure, the electrical and optical stimulation units 210, 220, with the light emitting diode 211 and the stimulation electrode 221 are arranged. Furthermore, the support plate 243 carries a power supply section 230, comprising e.g. a rechargeable accumulator. On the opposite site of the support plate 243, the electrical contacts 251 are arranged, which are exposed at the upper surface 241 of the enclosure 240 (see Figures 3A, 4A).

Figure 5B shows the plan view of the support plate 243 according to arrow B in Figure 5A. Accordingly, the support plate 243 carries the accumulator 231 and the stimulation units 210, 220. Furthermore, Figure 5C shows a plan view of the support plates 243 according to arrow C in Figure 5A, including the contacts 251.

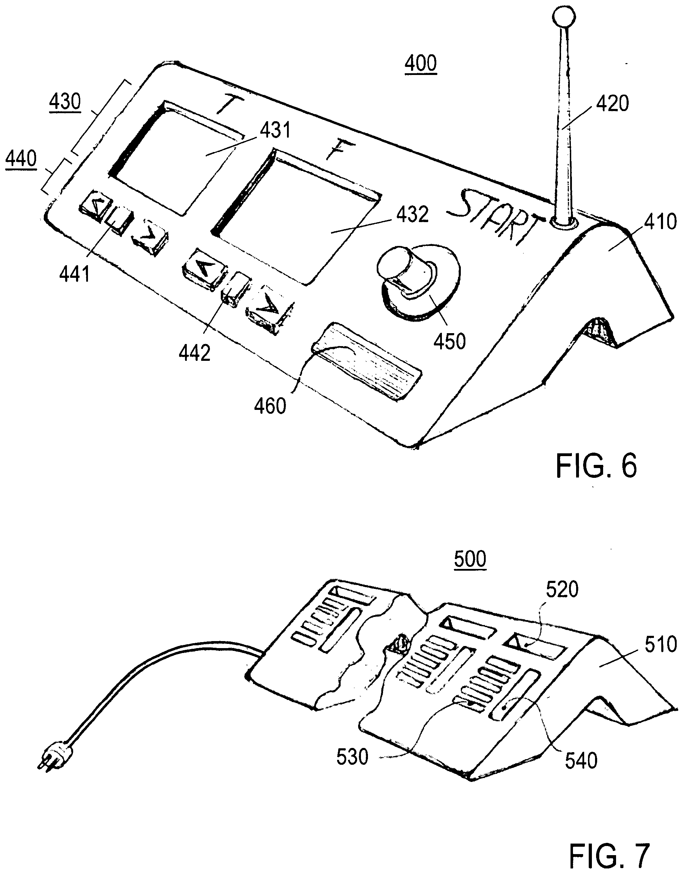

Figures 6 and 7 illustrate a main control 400 and a recharging device 500, which can be provided as separate apparatuses (as shown) or alternatively integrated into a common apparatus. The main control 400 of Figure 6 comprises a housing 410, a transmitter antenna 420, a display device 430 with a first display 431 and a second display 432, a selector device 440 with a first selector 441 and a second selector 442, a start switch 450 and an acoustic alarm device 460. The first display 431 shows a time program of the stimulation protocol selected with the first selector 441, and the second display 432 shows a frequency program of the stimulation protocol selected with the second selector 442. The acoustic alarm device 460 can be used for indicating the end of the stimulation procedure.

The charging device 500 of Figure 7 (partially shown) comprises a housing 510, a plurality of interface sections 520 (e. g. 8 or 12), a plurality of associated charging condition display devices 530 and a plurality of associated alarm display devices 540 (each for one of the interface sections 520). The interface sections 520 are adapted for electrically coupling and recharging stimulation devices 200. Preferably, the interfaces 520 have a shape being adapted to the outer shape of the enclosure 240 of the stimulation device 200. The charging can be implemented directly using the contacts 251 of the stimulation device 200 (see e.g. Figures 3A, 4A).

In practical use, an inventive stimulation system includes a plurality of treatment apparatuses 300 (according to Figure 2) and a main control 400, optionally with the integrated recharging device 500. The treatment apparatuses 300 are distributed and adherently fixed in a detachable manner on the body surface of a person to be treated. The time and frequency programme of the stimulation procedure is selected by a user with the first and second selectors 441, 442 of the main control 400. The operation of the treatment apparatuses 300 is activated by activating the start switch 450. Control signals are transmitted via the transmitter antenna 420 to the antenna devices 120 of the treatment apparatuses 300. The switching devices 140 close the contacts 251 of the stimulation devices 200 with the parameters of the selected time and frequency programs, thus starting the operation of the electrical and optical stimulation units 210, 220 according to the selected time and frequency programs. After the expiration of the stimulation time, an alarm signal is emitted from the acoustic alarm device 460. The treatment apparatuses 300 are separated from the body surface 1. Subsequently, the adhering components 110 of the positioning devices 100 are discarded, while the stimulation devices 200 are prepared for further use, e. g. using the recharging device 500.

The features of the invention in the above description, the drawings and the claims can be of significance individually or in combination or in sub-combination for the realization for the invention in its various embodiments.

## Claims

1. Positioning device (100), which is configured for positioning a stimulation device (200), in particular for low level laser therapy applications, on a body surface (1) of a person to be stimulated, comprising:
- an adhering component (110), which is adapted for detachably accommodating the stimulation device (200) and for a releasable arrangement on the body surface (1),
- an antenna device (120), which is fixedly connected with the adhering component (110), said antenna device (120) being arranged for receiving a control signal for controlling the stimulation device (200), and
- a carrier contact section (130), which is fixedly connected with the adhering component (110), said carrier contact section (130) including two contacts (131) and being arranged for providing an electrical contact with the stimulation device (200),
**characterized by**
- a switching device (140), which is fixedly connected with the adhering component (110) and electrically interconnected between the antenna device (120) and the carrier contact section (130),
- the switching device (140) being adapted for electrically connecting the contacts (131) in response to the control signal received by the antenna device (120).

2. Positioning device according to claim 1, wherein the adhering component (110) comprises
- at least one adhesive layer (111) being arranged for the contact with the body surface (1), and
- a carrier web (112), which carries the antenna device (120) and the switching device (140) and further is adapted to form a receptacle for accommodating the stimulation device (200).

3. Positioning device according to claim 2, wherein
- the antenna device (120) and the switching device (140) are arranged on an outer surface of the carrier web (112), and
- the carrier contact section (130) is arranged on an inner side of the receptacle provided by the carrier web (112).

4. Positioning device according to one of the foregoing claims, wherein
- when the stimulation device (200) is coupled with the positioning device (100), a length of an electrical connection between the antenna device (120) and the switching device (140) is shorter than a length of an electrical connection between the switching device (140) via the carrier contact section (130) to the stimulation device (200).

5. Positioning device according to one of the foregoing claims, wherein
- the adhering component (110) is a disposable component, in particular having the shape of an adhesive plaster.

6. Treatment apparatus (300), which is configured for a stimulation treatment of a person, comprising:
- the positioning device (100) according to one of the foregoing claims, and
- a stimulation device (200) comprising at least one stimulation unit (210, 220), a power supply section (230) for supplying electrical power to the at least one stimulation unit (210, 220), and a stimulation device contact section (250), wherein
- the stimulation device (200) is coupled with the positioning device (100), and
- the stimulation device contact section (250) is in contact with the carrier contact section (130) of the positioning device (100).

7. Treatment apparatus according to claim 6, wherein
- the at least one stimulation unit (210, 220) includes at least one of an optical stimulation unit (210) and an electrical stimulation unit (220).

8. Treatment apparatus according to claim 6 or 7, wherein
- the stimulation device (200) comprises an enclosure (240) accommodating the at least one stimulation unit (210, 220) and the power supply (230), wherein
- the enclosure (240) includes the stimulation device contact section (250), which is arranged for a contact with the carrier contact section (130) of the positioning device (100).

9. Treatment apparatus according to one of the claims 6 to 8, further comprising
- an anchoring section (260) of the stimulation device (200), wherein the anchoring section (260) is adapted for a releasable connection of the stimulation device (200) with the positioning device (100).

10. Treatment apparatus according to claim 9, wherein the anchoring section (260) includes at least one of the features
- the anchoring section (260) comprising spikes (261), which are arranged for a connection with the carrier web (112),
- the anchoring section (260) comprising receptacle grooves (262) for accommodating edges of the carrier web (112), and
- the anchoring section (260) being provided on an outer surface of the enclosure (240).

11. Stimulation system, comprising
- a plurality of treatment apparatuses (300) according to one of the claims 6 to 10, and
- a main control (400), which is adapted for creating control signals for controlling the treatment apparatuses (300).

12. Stimulation system according to claim 11, wherein
- the main control (400) further includes interface sections (520) for electrically coupling and recharging the stimulation devices (200) of the treatment apparatuses (300).

## Patentansprüche

1. Positioniereinrichtung (100), die zur Positionierung einer Stimulationseinrichtung (200), insbesondere für Niedriglevel-Lasertherapie-Anwendungen, auf einer Körperoberfläche (1) einer zu stimulierenden Person konfiguriert ist, umfassend:
- eine haftende Komponente (110), die zur lösbaren Aufnahme der Stimulationseinrichtung (200) und für eine freigebbare Anordnung auf der Körperoberfläche (1) angepasst ist,
- eine Antenneneinrichtung (120), die mit der haftende Komponente (110) fest verbunden ist, wobei die Antenneneinrichtung (120) zum Empfang eines Steuersignals zur Steuerung der Stimula-tionseinrichtung (200) angeordnet ist, und
- einen Trägerkontaktabschnitt (130), der fest mit der haftenden Komponente (110) verbunden ist, wobei der Trägerkontaktabschnitt (130) zwei Kontakte (131) enthält und zur Bereitstellung eines elektrischen Kontakts mit der Stimulationseinrichtung (200) angeordnet ist,
**gekennzeichnet durch**
- eine Schalteinrichtung (140), die fest mit der haftenden Komponente (110) verbunden und zwischen der Antenneneinrichtung (120) und dem Trägerkontaktabschnitt (130) elektrisch verbunden ist, wobei
- die Schalteinrichtung (140) zur elektrischen Verbindung der Kontakte (131) in Reaktion auf das Steuersignal ausgelegt ist, das durch die Antenneneinrichtung (120) empfangen wird.

2. Positioniereinrichtung gemäß Anspruch 1, wobei die haftende Komponente (110) umfasst
- mindestens eine adhäsive Schicht (111), die für den Kontakt mit der Körperoberfläche (1) angeordnet ist, und
- eine Trägerbahn (112), welche die Antenneneinrichtung (120) und die Schalteinrichtung (140) trägt und ferner angepasst ist, ein Behältnis zur Aufnahme der Stimulationseinrichtung (200) zu bilden.

3. Positioniereinrichtung gemäß Anspruch 2, wobei
- die Antenneneinrichtung (120) und die Schalteinrichtung (140) auf einer äußeren Oberfläche der Trägerbahn (112) angeordnet sind, und
- der Trägerkontaktabschnitt (130) auf der inneren Seite des Behältnisses angeordnet ist, das durch die Trägerbahn (112) bereitgestellt wird.

4. Positioniereinrichtung gemäß einem der vorhergehenden Ansprüche, wobei
- wenn die Stimulationseinrichtung (200) mit der Positioniereinrichtung (100) gekoppelt ist, eine Länge einer elektrischen Verbindung zwischen der Antenneneinrichtung (120) und der Schalteinrichtung (140) kürzer als eine Länge einer elektrischen Verbindung zwischen der Schalteinrichtung (140) über den Trägerkontaktabschnitt (130) zu der Stimulationseinrichtung (200) ist.

5. Positioniereinrichtung gemäß einem der vorhergehenden Ansprüche, wobei
- die haftende Komponente (110) eine Einweg-Komponente ist, die insbesondere die Gestalt eines adhäsiven Pflasters aufweist.

6. Behandlungsvorrichtung (300), die für eine Stimulationsbehandlung einer Person konfiguriert ist, umfassend:
- die Positioniereinrichtung (1) gemäß einem der vorhergehenden Ansprüche, und
- eine Stimulationseinrichtung (200), umfassend mindestens eine Stimulationseinheit (210, 220), einen Energieversorgungsabschnitt (230) zur Lieferung elektrischer Energie zu der mindestens einen Stimulationseinheit (210, 220), und einen Stimulationseinrichtung-Kontaktabschnitt (250), wobei
- die Stimulationseinrichtung (200) mit der Positioniereinrichtung (10) gekoppelt ist, und
- der Stimulationseinrichtungs-Kontaktabschnitt (250) in Kontakt mit dem Trägerkontaktabschnitt (130) der Positioniereinrichtung (100) ist.

7. Behandlungsvorrichtung gemäß Anspruch 6, wobei
- die mindestens eine Stimulationseinheit (210, 220) mindestens eine von einer optischen Stimulationseinheit (210) und einer elektrischen Stimulationseinheit (220) enthält.

8. Behandlungsvorrichtung gemäß Anspruch 6 oder 7, wobei
- die Stimulationseinrichtung (200) ein Gehäuse (240) umfasst, das die mindestens eine Stimulationseinheit (210, 220) und die Energieversorgung (230) aufnimmt, wobei
- das Gehäuse (240) den Stimulationseinrichtungs-Kontaktabschnitt (250) enthält, der für einen Kontakt mit dem Trägerkontaktabschnitt (130) der Positioniereinrichtung (100) angeordnet ist.

9. Behandlungsvorrichtung gemäß einem der Ansprüche 6 bis 8, ferner umfassend
- einen Ankerabschnitt (260) der Stimulationseinrichtung (200), wobei der Ankerabschnitt (260) für eine lösbare Verbindung der Stimulationseinrichtung (200) mit der Positioniereinrichtung (100) angeordnet ist.

10. Behandlungsvorrichtung gemäß Anspruch 9, wobei der Ankerabschnitt (260) mindestens eines der Merkmale enthält
- der Ankerabschnitt (260) umfasst Stachel (261), die für eine Verbindung mit der Trägerbahn (112) angeordnet sind,
- der Ankerabschnitt (260) umfasst Aufnahmenuten (262) zur Aufnahme von Kanten der Trägerbahn (112), und
- der Ankerabschnitt (260) ist auf einer äußeren Oberfläche des Gehäuses (240) bereitgestellt.

11. Stimulationssystem, umfassend
- eine Vielzahl von Behandlungsvorrichtungen (300) gemäß einem der Ansprüche 6 bis 10, und
- eine Hauptsteuerung (400), die zur Erzeugung von Steuersignalen zur Steuerung der Behandlungsvorrichtungen (300) angepasst ist.

12. Stimulationssystem gemäß Anspruch 11, wobei
- die Hauptsteuerung (400) ferner Schnittstellenabschnitte (520) zur elektrischen Kopplung und Wiederaufladung der Stimulationseinrichtungen (200) der Behandlungsvorrichtungen (300) enthält.

## Revendications

1. Dispositif de positionnement (100), qui est configuré pour positionner un dispositif de stimulation (200), en particulier pour des applications de thérapie au laser de faible niveau, sur une surface corporelle (1) d'une personne à stimuler, comprenant :
- un composant adhérant (110), qui est adapté pour accueillir de manière détachable le dispositif de stimulation (200) et pour l'agencement amovible sur la surface corporelle (1),
- un dispositif d'antenne (120), qui est connecté à demeure au composant adhérant (110), ledit dispositif d'antenne (120) étant agencé pour recevoir un signal de commande pour commander le dispositif de stimulation (200), et
- une section de contact porteur (130), qui est connectée à demeure au composant adhérant (110), lesdites sections de contact porteur (130) incluant deux contacts (131) et étant agencées pour fournir un contact électrique avec le dispositif de stimulation (200),
**caractérisé par**
- un dispositif de commutation (140), qui est connecté à demeure au composant adhérant (110) et interconnecté électriquement entre le dispositif d'antenne (120) et la section de contact porteur (130),
- le dispositif de commutation (140) étant adapté pour connecter électriquement les contacts (131) en réponse au signal de commande reçu par le dispositif d'antenne (120).

2. Dispositif de positionnement selon la revendication 1, dans lequel le composant adhérant (110) comprend
- au moins une couche adhésive (111) étant agencée pour le contact avec la surface corporelle (1), et
- une bande porteuse (112), qui porte le dispositif d'antenne (120) et le dispositif de commutation (140) et est en outre adaptée pour former un réceptacle pour accueillir le dispositif de stimulation (200).

3. Dispositif de positionnement selon la revendication 2, dans lequel
- le dispositif d'antenne (120) et le dispositif de commutation (140) sont agencés sur une surface extérieure de la bande porteuse (112), et
- la section de contact porteur (130) est agencée sur un côté intérieur du réceptacle fourni par la bande porteuse (112).

4. Dispositif de positionnement selon l'une des revendications précédentes, dans lequel
- quand le dispositif de stimulation (200) est couplé avec le dispositif de positionnement (100), une longueur d'une connexion électrique entre le dispositif d'antenne (120) et le dispositif de commutation (140) est plus courte qu'une longueur d'une connexion électrique entre le dispositif de commutation (140) via la section de contact porteur (130) jusqu'au dispositif de stimulation (200).

5. Dispositif de positionnement selon l'une des revendications précédentes, dans lequel
- le composant adhérant (110) est un composant jetable, en particulier ayant la forme d'un emplâtre adhésif.

6. Appareil de traitement (300), qui est configuré pour un traitement de stimulation d'une personne, comprenant :
- le dispositif de positionnement (100) selon l'une des revendications précédentes, et
- un dispositif de stimulation (200) comprenant au moins une unité de stimulation (210, 220), une section d'alimentation (230) pour apporter de l'énergie électrique à l'au moins une unité de stimulation (210, 220), et une section de contact de dispositif de stimulation (250), dans lequel
- le dispositif de stimulation (200) est couplé au dispositif de positionnement (100), et
- la section de contact de dispositif de stimulation (250) est en contact avec la section de contact porteur (130) du dispositif de positionnement (100).

7. Appareil de traitement selon la revendication 6, dans lequel
- l'au moins une unité de stimulation (210, 220) inclut au moins une parmi une unité de stimulation optique (210) et une unité de stimulation électrique (220).

8. Appareil de traitement selon la revendication 6 ou 7, dans lequel
- le dispositif de stimulation (200) comprenant une enceinte (240) accueillant l'au moins une unité de stimulation (210, 220) et l'alimentation (230), dans lequel
- l'enceinte (240) inclut la section de contact de dispositif de stimulation (250), qui est agencée pour un contact avec la section de contact porteur (130) du dispositif de positionnement (100).

9. Appareil de traitement selon l'une des revendications 6 à 8, comprenant en outre
- une section d'ancrage (260) du dispositif de stimulation (200), dans lequel la section d'ancrage (260) est adaptée pour une connexion amovible du dispositif de stimulation (200) au dispositif de positionnement (100).

10. Appareil de traitement selon la revendication 9, dans lequel la section d'ancrage (260) inclut au moins l'une des caractéristiques
- la section d'ancrage (260) comprenant des pointes (261), qui sont agencées pour une connexion à la bande porteuse (112),
- la section d'ancrage (260) comprenant des rainures de réceptacle (262) pour accueillir des bords de la bande porteuse (112), et
- la section d'ancrage (260) étant disposée sur une surface extérieure de l'enceinte (240).

11. Système de stimulation, comprenant
- une pluralité d'appareils de traitement (300) selon l'une des revendications 6 à 10, et
- une commande principale (400), qui est adaptée pour créer des signaux de commande pour commander les appareils de traitement (300).

12. Système de stimulation selon la revendication 11, dans lequel
- la commande principale (400) inclut en outre des sections d'interface (520) pour coupler électriquement et recharger les dispositifs de stimulation (200) des appareils de traitement (300).
